# EUROPEAN PATENT APPLICATION

(11) **EP 3 308 717 A1**
(43) Date of publication of application: **18.04.2018**
(21) Application number: 16807305.4
(22) Date of filing: 27.05.2016
(51) Int. Cl.: A61B 10/02

(54) **BIOPSY NEEDLE**

(30) Priority: 10.06.2015 JP 2015117470
(71) Applicant: Olympus Corporation, Hachioji-shi Tokyo 192-8507 (JP)
(72) Inventor: NISHINA, Kenichi, Tokyo 192-8507 (JP); IIJIMA, Yasuhiro, Tokyo 192-8507 (JP)
(74) Representative: Schicker, Silvia
(86) International application number: PCT/JP2016/065699
(87) International publication number: WO 2016/199598

(57) **Abstract**

A biopsy needle (100) according to the present invention includes: a tubular outer needle (110) having a needle tip (111) at one end in a longitudinal direction of the outer needle (110); and an inner needle (120) having a cylindrical shape and configured to be inserted into the outer needle (110) and movable along the longitudinal direction of the outer needle (110). The inner needle (120) includes a needle tip (121) at a distal end of the inner needle and includes concave portions (123A, 123B) provided on first and second side surfaces of the inner needle, the first side surface facing a first direction perpendicular to a cross section parallel to the longitudinal direction, the second side surface facing a second direction opposite to the first direction.

## Description

### Field

The present invention relates to a biopsy needle for collecting body tissues.

### Background

Conventionally, a biopsy is performed for pathological definitive diagnosis or the like by inducing a narrow and long biopsy needle to an observed region through a treatment tool channel of an ultrasound endoscope using an ultrasound tomogram obtained by the ultrasound endoscope as a guide, and puncturing a lesion tissue to collect body tissues. As a biopsy needle, proposed is a configuration in which an inner needle with a cutout (notch) for tissue collection formed in a side surface at a distal end side is arranged inside a hollow outer needle, and the inner needle and the outer needle are movable by a moving mechanism (for example, see Patent Literature 1). During biopsy, the biopsy needle is caused to puncture up to tissues as a biopsy region in a state where the outer needle of the biopsy needle substantially covers the notch of the inner needle, and then, the inner needle is caused to protrude from a distal end of the outer needle until the notch is exposed. Accordingly, the body tissue enters an inside of the notch, and thus, the body tissue which has entered the inside of the notch is cut by the distal end of the outer needle by advancing the outer needle, and the notch is covered by the outer needle in a state where the body tissue is secured inside the notch. After pulling out the biopsy needle from the biopsy region in such a state, the notch is exposed by retracting the outer needle, thereby collecting the body tissues inside the notch.

### Citation List

### Patent Literature

Patent Literature 1: JP-T 2009-531115

### Summary

### Technical Problem

Biopsy for diagnosis of prostate cancer is currently performed in a transrectal or transperineal manner, but there is a demand for a method of performing biopsy from an inside of prostate in a transurethral manner in order to reduce invasion to a patient. According to this method, there is a possibility that it is possible to further reduce a patient burden without causing a needle tip to contact a nerve traveling an outer side of the prostate as long as a capsule (outer membrane) of the prostate is not penetrated outwardly from the inner side.

However, the notch that enables collection of a specimen is a portion at a proximal end side separated from the needle tip by about 5 mm in the conventional biopsy needle. Thus, it is considered even a case where the capsule is penetrated by the needle tip by causing the notch to arrive closely at a capsule boundary in order to collect tissues present closely to the capsule boundary of the prostate, and accordingly, it is difficult to deny a possibility that the needle tip contacts the nerve. Thus, there is a request for a configuration which enables collection of the tissue in the vicinity of the capsule boundary almost without causing the needle tip of the biopsy needle to move outside the capsule from the capsule of the prostate.

The present invention has been made in view of the foregoing, and an object of the invention is to provide a biopsy needle which is capable of reliably collecting tissues and shortening a protruding length of the biopsy needle that protrudes from a collection target region in collecting the tissues.

### Solution to Problem

In order to solve the above described problem and achieve the object, a biopsy needle includes: an outer needle having a tubular shape and including a first needle tip at one end in a longitudinal direction of the outer needle; and an inner needle having a columnar shape and configured to be inserted into the outer needle and movable along the longitudinal direction. The inner needle includes: a second needle tip at a distal end of the inner needle; and concave portions provided on first and second side surfaces of the inner needle, the first side surface facing a first direction perpendicular to a cross section parallel to the longitudinal direction, the second side surface facing a second direction opposite to the first direction.

In the biopsy needle according to the invention, the cross section passes through the second needle tip.

In the biopsy needle according to the invention, the cross section passes through a central axis in the longitudinal direction of the inner needle.

In the biopsy needle according to the invention, the inner needle further includes an inclined surface that is inclined toward the second needle tip, and a part of the inclined surface is included in an area obtained by projecting the concave portions in a direction parallel to a transverse direction of the inner needle.

In the biopsy needle according to the invention, a width in a radial direction of each of the concave portions is narrower at a distal end side than at a proximal end side.

In the biopsy needle according to the invention, the inner needle further includes a groove leading from the inclined surface to at least one of the concave portions.

In the biopsy needle according to the invention, the inner needle further includes a cutout portion in which a part of the inner needle between the two concave portions is cut out in the longitudinal direction to link the two concave portions with each other.

In the biopsy needle according to the invention, the cutout portion is located on a same side as the second needle tip in a transverse direction of the inner needle.

In the biopsy needle according to the invention, the cutout portion is located on an opposite side to the second needle tip in a transverse direction of the inner needle.

The biopsy needle according to the invention further includes a moving mechanism configured to cause the outer needle and the inner needle to independently slide in the longitudinal direction, a maximum slidable distance of the outer needle in a distal end direction being larger than a maximum slidable distance of the inner needle in the distal end direction. The moving mechanism is configured to move the outer needle and the inner needle so as to switch between a first state where the second needle tip is positioned closer to a distal end side than the first needle tip and a second state where the first needle tip is positioned closer to the distal end side than the second needle tip.

In the biopsy needle according to the invention, the inner needle has a cylindrical shape.

### Advantageous Effects of Invention

A biopsy needle according to the present invention includes: an outer needle having a tubular shape and including a first needle tip at one end in a longitudinal direction of the outer needle; and an inner needle having a columnar shape and configured to be inserted into the outer needle and movable along the longitudinal direction. The inner needle includes a second needle tip at a distal end of the inner needle, and concave portions provided on first and second side surfaces of the inner needle, the first side surface facing a first direction perpendicular to a cross section parallel to the longitudinal direction, the second side surface facing a second direction opposite to the first direction. With this structure, it is possible to reliably collect tissues into the concave portions of the inner needle and to shorten a protruding length of the biopsy needle protruding from a collection target region in the tissues by adjusting a location in the concave portions of the inner needle. Brief Description of Drawings

FIG. 1 is a schematic view illustrating a distal end portion of a biopsy needle according to a first embodiment of the present invention.
FIG. 2 is a schematic view seen along an arrow A of FIG. 1.
FIG. 3 is a cross sectional view taken along a line B-B of FIG. 1.
FIG. 4 is a schematic view illustrating a biopsy method according to the biopsy needle illustrated in FIG. 1.
FIG. 5A is a cross sectional view of a distal end of a conventional biopsy needle taken along a plane passing through a central axis in a longitudinal direction of the biopsy needle and a center of a needle tip of an inner needle.
FIG. 5B is a cross sectional view of the conventional biopsy needle taken along a line R-R of FIG. 5A.
FIG. 6 is a cross sectional view of a distal end of a conventional biopsy needle taken along a plane passing through a central axis in a longitudinal direction of the biopsy needle and a center of a needle tip of an inner needle.
FIG. 7 is a cross sectional view of the biopsy needle according to the first embodiment taken along a plane passing through a central axis in a longitudinal direction of the biopsy needle.
FIG. 8 is a schematic view illustrating a first state and a second state of the biopsy needle illustrated in FIG. 1.
FIG. 9 is a schematic view illustrating protruding operations of an outer needle and the inner needle of the biopsy needle illustrated in FIG. 1.
FIG. 10A is a schematic view illustrating a biopsy operation of the biopsy needle illustrated in FIG. 7.
FIG. 10B is a schematic view illustrating a biopsy operation of the biopsy needle illustrated in FIG. 7.
FIG. 10C is a schematic view illustrating a biopsy operation of the biopsy needle illustrated in FIG. 7.
FIG. 10D is a schematic view illustrating a biopsy operation of the biopsy needle illustrated in FIG. 7.
FIG. 10E is a schematic view illustrating a biopsy operation of the biopsy needle illustrated in FIG. 7.
FIG. 10F is a schematic view illustrating a biopsy operation of the biopsy needle illustrated in FIG. 7.
FIG. 11 is a schematic view illustrating a distal end portion of a biopsy needle according to a modified example of the first embodiment.
FIG. 12 is a perspective view of a distal end portion of an inner needle illustrated in FIG. 11.
FIG. 13 is a schematic view seen along an arrow C of FIG. 11.
FIG. 14 is a cross sectional view taken along a line D-D of FIG. 11.
FIG. 15 is a schematic view illustrating a distal end portion of a biopsy needle according to a second embodiment.
FIG. 16 is a schematic view seen along an arrow E of FIG. 15.
FIG. 17 is a cross sectional view taken along a line F-F of FIG. 15.
FIG. 18 is a schematic view illustrating another example of the distal end portion of the biopsy needle according to the second embodiment.
FIG. 19 is a schematic view seen along an arrow G of FIG. 18.
FIG. 20 is a cross sectional view taken along a line H-H of FIG. 18.
FIG. 21 is a schematic view illustrating a distal end portion of a biopsy needle according to a third embodiment.
FIG. 22 is a schematic view seen along an arrow I of FIG. 21.
FIG. 23 is a cross sectional view taken along a line J-J of FIG. 21.
FIG. 24 is a schematic view illustrating a distal end portion of a biopsy needle according to a fourth embodiment.
FIG. 25 is a schematic view seen along an arrow K of FIG. 24.
FIG. 26 is a cross sectional view taken along a line L-L of FIG. 24.
FIG. 27 is a cross sectional view taken along a line M-M of FIG. 24.

### Description of Embodiments

Hereinafter, modes for carrying out the present invention will be described in detail with reference to the drawings. The present invention is not limited to the following embodiments. The drawings referred to in the following description schematically illustrate shapes, sizes, and positional relationships merely to make contents of the present invention understandable. That is, the present invention is not limited only to the shapes, sizes, and positional relationships shown in the respective drawings. Reference will be made below to an exemplary biopsy needle for puncturing into body tissues of an animal including human to collect the body tissues. However, the present invention is not limited to the embodiments. The same reference signs are used to designate the same elements throughout the drawings.

### (First Embodiment)

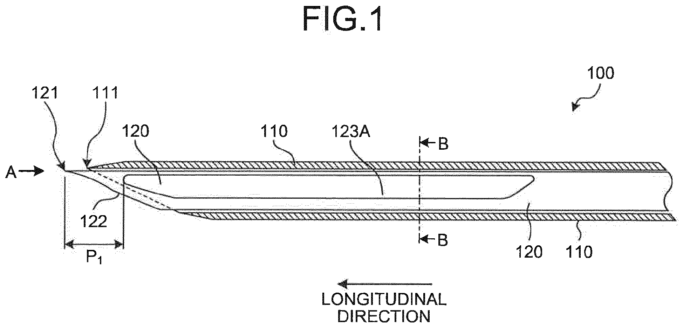

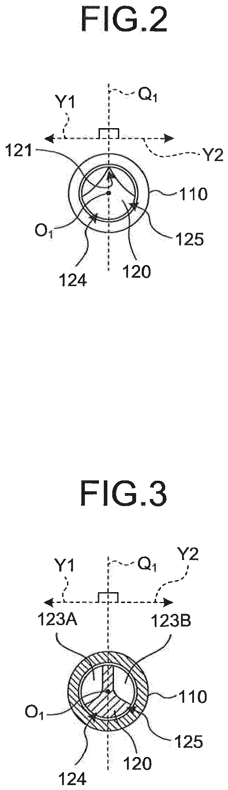

FIG. 1 is a schematic view illustrating a distal end portion of a biopsy needle according to a first embodiment. For illustrative purposes, FIG. 1 shows a cross sectional view of an outer needle taken along a plane passing through a central axis in a longitudinal direction of the outer needle. FIG. 2 is a schematic view seen along an arrow A of FIG. 1. FIG. 3 is a cross sectional view taken along a line B-B of FIG. 1.

As illustrated in FIGS. 1 to 3, a biopsy needle 100 according to the first embodiment includes a tubular outer needle 110 extending in the longitudinal direction and a cylindrical-shaped inner needle 120 which is solid and is inserted into the outer needle 110 and movable along the longitudinal direction. The inner needle 120 may have a prismatic shape instead of the cylindrical shape. The biopsy needle 100 further includes an operating unit (not illustrated) which houses each proximal end of the outer needle 110 and the inner needle 120 therein and includes a moving mechanism that causes each of the outer needle 110 and the inner needle 120 to independently slide in the longitudinal direction as will be described below.

The outer needle 110 has the tubular shape with a sharp distal end and includes a needle tip 111 (first needle tip) at the distal end in the longitudinal direction. The outer needle 110 is formed using a material having biocompatibility, for example, metal such as stainless, titanium, and aluminum or resin such as fluororesin.

The inner needle 120 is formed using a material having biocompatibility, similar to the outer needle 110, and includes a needle tip 121 (second needle tip) formed at a distal end and an inclined surface 122 which is inclined toward the needle tip 121 at the distal end.

A cross section Q₁ passing through a central axis O₁ in the longitudinal direction of the inner needle 120 passes through the needle tip 121 (see FIG. 2). When seen in a transverse direction, the distal end of the inner needle 120 is cut such that an apex thereof is formed at the opposite side to the inclined surface 122, thereby sharpening the needle tip 121. The inclined surface 122 of the distal end of the inner needle 120 is formed using a processing method such as lancet, back-cut, semi-lancet, and flat-sharpening.

Further, when a direction vertical to the cross section Q₁ is defined as a first direction Y1 (see FIG. 2) and an opposite direction to the first direction Y1 is defined as a second direction Y2, the inner needle 120 includes two concave portions 123A and 123B provided at sides of both side surface of a first side surface 124 in the first direction Y1 and a second side surface 125 in the second direction Y2. The two concave portions 123A and 123B are provided to collect body tissues in the side surface of the inner needle 120. The concave portions 123A and 123B are provided at both sides of the cross section Q₁ (see FIG. 3.). In the example of FIG. 3, the concave portions 123A and 123B are bilaterally symmetrical with respect to the cross section Q₁. The inner needle 120 has a shape in which the concave portions 123A and 123B are bilaterally symmetrical from the side surface of the solid cylindrical-shaped inner needle 120, and a portion between the concave portions 123A and 123B is left along the longitudinal direction.

A depth of each of the concave portions 123A and 123B is gradually shallower toward the needle tip 121. Further, a width in a radial direction of each of the concave portions 123A and 123B is narrower at the distal end side than at the proximal end side, and the concave portions 123A and 123B are set such that each width gets narrower toward the needle tip 121. When each shape of the concave portions 123A and 123B is set in this manner, each distal end of the concave portions 123A and 123B reaches up to a location of the inclined surface 122. That is, a part of the inclined surface 122 is included in an area obtained by projecting the concave portions 123A and 123B to be parallel to the transverse direction of the inner needle 120. A distance P₁ between the needle tip 121 of the inner needle 120 and each distal end of the concave portions 123A and 123B is narrowed up to about 1.5 mm in the case of the biopsy needle 100 of 18 gauge. As a result, body tissues in the vicinity of the needle tip 121 can be collected into the concave portions 123A and 123B.

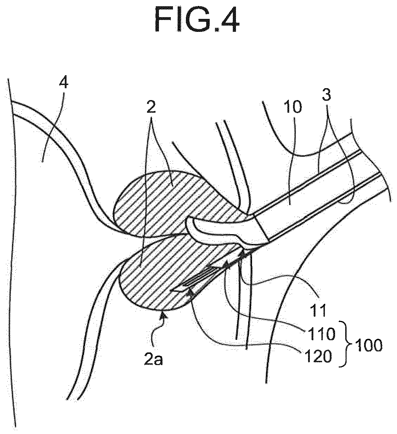

FIG. 4 is a schematic view illustrating a biopsy method according to the biopsy needle 100 illustrated in FIG. 1. As illustrated in FIG. 4, an insertion portion 10 of an ultrasound endoscope is caused to reach up to a prostate 2 as a biopsy target via a urethra 3 of a patient using an ultrasound tomogram obtained by the ultrasound endoscope as a guide. A distal end of the biopsy needle inserted into a treatment tool channel of the insertion portion 10 is caused to protrude from an aperture portion 11 at a distal end of the insertion portion 10 so that the outer needle 110 and the inner needle 120 puncture the prostate 2. The outer needle 110 is caused to advance in a distal end direction after causing the distal end of the inner needle 120 to protrude from the outer needle 110 until the concave portions 123A and 123B are exposed, and the concave portions 123A and 123B is covered by the outer needle 110 in a state where the body tissue is secured inside the concave portions 123A and 123B while cutting the body tissue which has entered each inside of the concave portions 123A and 123B by the distal end of the outer needle 110. In this state, the biopsy needle 100 is pulled out via the treatment tool channel outside the body, and then, the body tissue secured inside the concave portions 123A and 123B are collected. A bladder 4 is positioned inside the prostate 2.

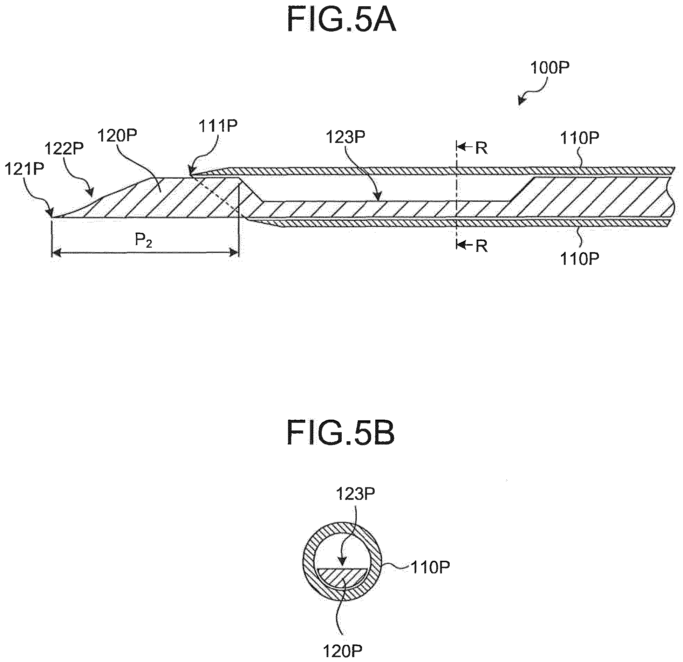

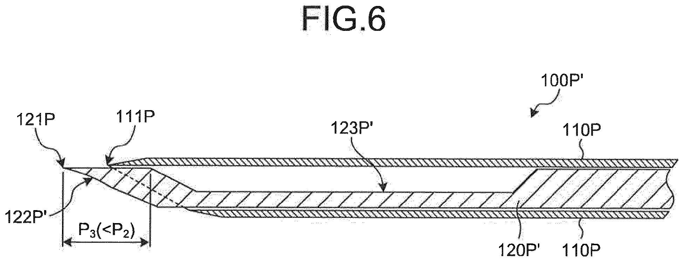

Here, conventional biopsy needles will be described. FIGS. 5A and 6 are cross sectional views of a distal end of the conventional biopsy needle taken along a plane passing through a central axis in a longitudinal direction of the biopsy needle and a center of a needle tip of an inner needle. FIG. 5B is a cross sectional view of the conventional biopsy needle taken along a line R-R of FIG. 5A. As illustrated in FIGS. 5A and 5B, a notch portion 123P, obtained by cutting out a side surface at a distal end side of an inner needle 120P such that a cross section thereof becomes a semicircular shape, is formed in a conventional biopsy needle 100P to collect body tissues. In this configuration, a length P₂ from a needle tip 121P of the inner needle 120P to the notch portion 123P is lengthened up to a length of about 5 to 6 mm to set a certain margin in order to reliably cover even a distal end of the notch portion 123P with an outer needle 110P such that the body tissue does not escape from the notch portion 123P after collection of the body tissue. Thus, the conventional biopsy needle 100P is forced to cause the needle tip 121P of the inner needle 120P to deeply puncture up to a depth deeper than a region where tissues to be collected are present by 5 to 6 mm. For this reason, there is also a study for a configuration in which a notch portion 123P' is stretched in a distal end direction up to the vicinity of a proximal end of an inclined surface 122P' and a length P₃ of an inner needle 120P' protruding from a collection target region in tissues is shortened up to about 2 to 3 mm, as in a biopsy needle 100P' illustrated in FIG. 6, in order to perform tissue collection without deeply inserting the needle into the tissue. However, the notch portion 123P' is stretched to the vicinity of the proximal end of the inclined surface 122P' in the case of the configuration illustrated in FIG. 6, and thus, there is a possibility that strength of a distal end portion of the inner needle 120P' decreases so that the inner needle 120P' is bent when the inner needle 120P' is caused to puncture a hard tissue.

In regard to this, the biopsy needle 100 according to the embodiment has the shape in which the concave portions 123A and 123B are bilaterally symmetrical from the side surface of the solid cylindrical-shaped inner needle 120. Accordingly, the portion between the concave portions 123A and 123B is left along the longitudinal direction, and thus, it is possible to secure the strength at the distal end side of the inner needle 120, a puncturing force with respect to the needle tip 121 is easily transmitted, and it is possible to lower the possibility that the inner needle 120 is bent at the time of causing the needle tip 121 to puncture a hard tissue.

In addition, a part of the inclined surface 122 is included in the area obtained by projecting the concave portions 123A and 123B in a direction parallel to the transverse direction of the inner needle 120 in the first embodiment. In other words, since the distal ends of the concave portions 123A and 123B are stretched up to the vicinity of the needle tip 121 in the first embodiment, it is possible to collect the body tissues in the vicinity of the needle tip 121 inside the concave portions 123A and 123B while shortening the protruding length of the inner needle 120 during the biopsy.

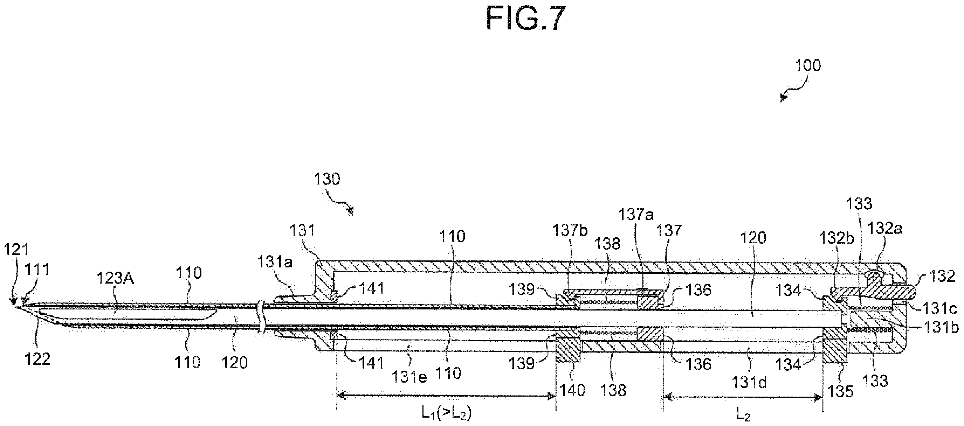

Next, the entire configuration of the biopsy needle 100 will be described. FIG. 7 is a cross sectional view of the biopsy needle 100 taken along the plane passing through the central axis in the longitudinal direction of the biopsy needle 100. For illustrative purposes, FIG. 7 shows the side surface of the inner needle 120 instead of the cross section thereof. As illustrated in FIG. 7, the biopsy needle 100 further includes an operating unit 130 which houses the proximal ends of the outer needle 110 and the inner needle 120 therein and includes the moving mechanism that independently moves each of the outer needle 110 and the inner needle 120 to be slidable in the longitudinal direction, in addition to the outer needle 110 and the inner needle 120.

The operating unit 130 has a configuration in which a trigger button 132, an inner needle charge coil spring 133, an inner needle slider 134, an inner needle knob 135, an inner needle stopper 136, an outer needle fixing hook releasing lever 137, an outer needle charge coil spring 138, an outer needle slider 139, an outer needle knob 140, and an outer needle stopper 141, which function as the moving mechanism, are assembled with an operating unit body 131 which has a hollow columnar shape.

In the operating unit body 131, a lure pipe sleeve portion 131a is provided at a distal end, a spring assembly convex portion 131b is provided at an internal proximal end, a trigger button hole 131c is provided in an upper portion of the spring assembly convex portion 131b, an inner needle knob hole 131d dug in the longitudinal direction is provided in a bottom portion at a proximal end side, and an outer needle knob hole 131e dug in the longitudinal direction is provided in a bottom portion at the distal end side. The distal ends of the outer needle 110 and the inner needle 120 protrude in the distal end direction from the lure pipe sleeve portion 131a. A proximal end of the inner needle charge coil spring 133 to be described later is assembled with the spring assembly convex portion 131b. A proximal end of the trigger button 132 to be described later protrude from the trigger button hole 131c. The inner needle knob 135 to be described later is slidable in the longitudinal direction inside the inner needle knob hole 131d. The outer needle knob 140 to be described later is slidable in the longitudinal direction inside the outer needle knob hole 131e.

The trigger button 132 serves as a trigger of an advancing operation of the inner needle 120, and both ends thereof move alternately up and down with a fulcrum 132a connected to the inside of the operating unit body 131 as an axis. An inner needle fixing hook 132b to be caught by a concave portion of the inner needle slider 134 to be described later is provided at a distal end of the trigger button 132.

The inner needle charge coil spring 133 has a proximal end assembled with a proximal end portion of the spring assembly convex portion 131b and a distal end assembled with a side surface at a proximal end side of the inner needle slider 134 to be described later, and biases the inner needle slider 134 toward the distal end direction by being extended after compression (charge).

The inner needle slider 134 is connected to the proximal end of the inner needle 120 and advances in the distal end direction by being biased toward the distal end direction by the inner needle charge coil spring 133, and accordingly, causes the inner needle 120 to advance in the distal end direction. The concave portion to which the inner needle fixing hook 132b is fitted is formed at a top surface of the inner needle slider 134.

The inner needle knob 135 slides along the inner needle knob hole 131d to be capable of advancing and retracting in the longitudinal direction. A top surface of the inner needle knob 135 is connected to a bottom surface of the inner needle slider 134. A lower portion of the inner needle knob 135 protrudes from the inner needle knob hole 131d. The inner needle knob 135 advances inside the inner needle knob hole 131d along with advancing of the inner needle slider 134. In addition, an operator of the biopsy needle 100 can retract the inner needle slider 134 and the inner needle 120 to the proximal end side by retracting the inner needle knob 135 to the proximal end side along the inner needle knob hole 131d. When the inner needle knob 135 retracts even to the proximal end of the inner needle knob hole 131d, the inner needle fixing hook 132b is fitted into the concave portion of the inner needle slider 134, and the inner needle 120 is fixed at a position arranged at the most proximal end side.

The inner needle stopper 136 stops the advancing operation of the inner needle slider 134, and accordingly, the advancing operation of the inner needle 120 is also stopped.

Both ends of the outer needle fixing hook releasing lever 137 move up and down with a fulcrum 137a connected to the inner needle stopper 136 as an axis. An outer needle fixing hook 137b to be caught by a concave portion of the outer needle slider 139 to be described later is provided at a distal end of the outer needle fixing hook releasing lever 137.

The outer needle charge coil spring 138 has a proximal end assembled with a side surface at a distal end side of the inner needle stopper 136 and a distal end assembled with a side surface at a proximal end side of the outer needle slider 139 to be described later, and biases the outer needle slider 139 toward the distal end direction by being extended after compression (charge).

The outer needle slider 139 is connected to the proximal end of the outer needle 110 and advances in the distal end direction by being biased toward the distal end direction by the outer needle charge coil spring 138, and accordingly, causes the outer needle 110 to advance in the distal end direction. The concave portion to which the outer needle fixing hook 137b is fitted is formed at a top surface of the outer needle slider 139.

The outer needle knob 140 slides along the outer needle knob hole 131e to be capable of advancing and retracting in the longitudinal direction. A top surface of the outer needle knob 140 is connected to a bottom surface of the outer needle slider 139. A lower portion of the outer needle knob 140 protrudes from the outer needle knob hole 131e. The outer needle knob 140 advances inside the outer needle knob hole 131e along with advancing of the outer needle slider 139. In addition, the operator of the biopsy needle 100 can retract the outer needle slider 139 and the outer needle 110 to the proximal end side by retracting the outer needle knob 140 to the proximal end side along the outer needle knob hole 131e. When the outer needle knob 140 retracts even to the proximal end of the outer needle knob hole 131e, the outer needle fixing hook 137b is fitted into the concave portion of the outer needle slider 139, and the outer needle 110 is fixed at a position arranged at the most proximal end side.

The outer needle stopper 141 is provided at a distal end portion of the operating unit body 131, stops the advancing operation of the outer needle slider 139, and accordingly, the advancing operation of the outer needle 110 is also stopped.

A length L₁ in the longitudinal direction of the outer needle knob hole 131e, that is, a stroke (maximum slidable distance) L₁ in distal end direction of the outer needle 110 is set to be longer than a length L₂ toward the longitudinal direction of the inner needle knob hole 131d, that is, the stroke L₂ toward the distal end direction of the inner needle 120. The stroke L₂ is set such that the concave portions 123A and 123B can be exposed from the needle tip 111 of the outer needle 110 to a degree of enabling the tissue collection.

Herein, the protruding length of the inner needle 120 from a collection target during the biopsy is reliably shortened such that the needle tip 121 of the inner needle 120 does not outwardly penetrate a capsule 2a of the prostate 2 from the inner side thereof, and the body tissues can be collected in the concave portions 123A and 123B by providing the concave portions 123A and 123B to be stretched up to the distal end side of the inner needle 120 and regulating a first state and a second state for switching between the first state and the second state, in the biopsy needle 100 according to the embodiment. The biopsy needle 100 regulates the first state and the second state by setting the stroke L₁ in the longitudinal direction of the outer needle 110 to be larger than the stroke L₂ the longitudinal direction of the inner needle 120. Next, the first state and the second state in the biopsy needle 100 will be described.

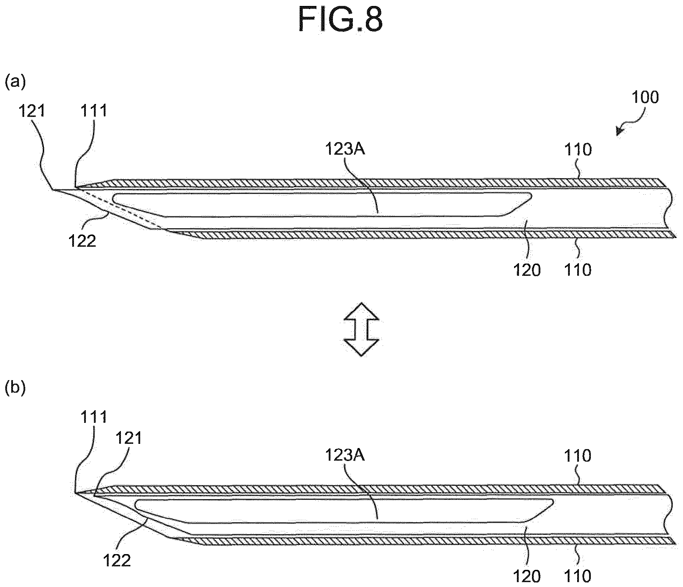

FIG. 8 is a schematic view illustrating the first state and second state of the biopsy needle 100. For illustrative purposes, FIG. 8 shows a cross sectional view of the outer needle 110 taken along the plane passing through the central axis in the longitudinal direction of the outer needle 110. In FIG. 8, (a) is a schematic view illustrating the first state. The first state is a charge state where both the inner needle charge coil spring 133 and the outer needle charge coil spring 138 are compressed and charged with energy for extending. In other words, the first state is a state where the outer needle 110 and the inner needle 120 are charged with energy for protruding, and is a state obtained when the outer needle 110 and the inner needle 120 puncture a biopsy region. As illustrated in (a) of FIG. 8, the needle tip 121 of the inner needle 120 are positioned more closely at the distal end side than the needle tip 111 of the outer needle 110 in the first state. In addition, it is ideal that the needle tip 111 of the outer needle 110 are positioned more closely at the distal end side than each distal end side of the concave portions 123A and 123B such that body tissues other than a collection target does not enter each inside of the concave portions 123A and 123B in the middle of reaching the body tissue as the collection target in the first state. For example, the distance P₁ between the needle tip 121 of the inner needle 120 and each distal end of the concave portions 123A and 123B is narrowed up to about 1.5 mm in the case of the biopsy needle 100 of 18 gauge.

In FIG. 8, (b) is a schematic view illustrating the second state of the biopsy needle 100, and is the cross sectional view of the distal end of the biopsy needle 100 taken along the plane passing through the central axis in the longitudinal direction and a center of the needle tip 121 of the inner needle 120. The second state is a state where the inner needle 120 and the outer needle 110 slide up to the biopsy region in the distal end direction and completes the biopsy collection. In the second state, the needle tip 111 of the outer needle 110 is positioned more closely at the distal end side than the needle tip 121 of the inner needle 120. That is, the outer needle 110 is turned into the state of entirely covering the concave portions 123A and 123B of the inner needle 120.

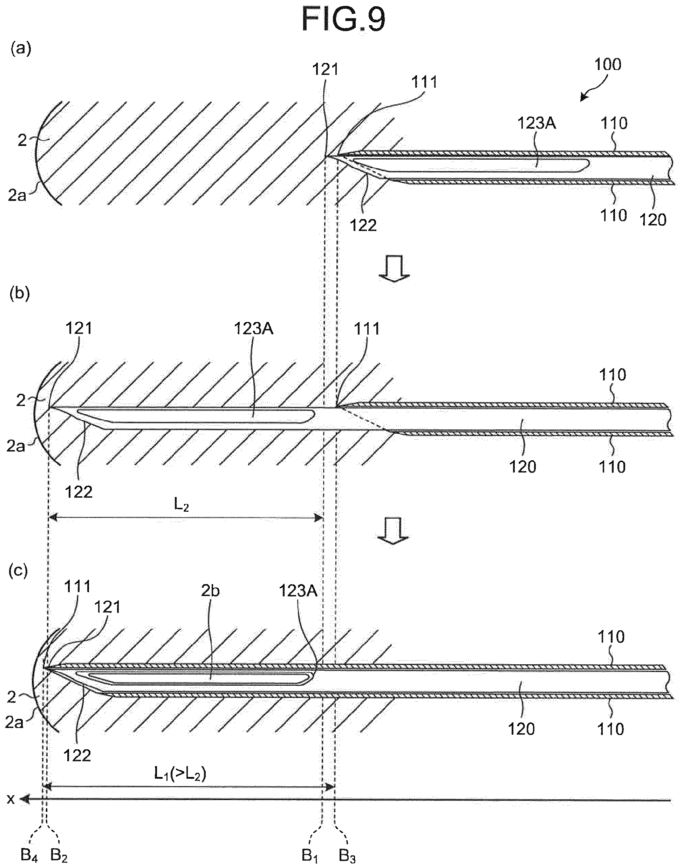

Next, FIG. 9 is a schematic view illustrating protruding operations of the outer needle 110 and the inner needle 120 of the biopsy needle 100. FIG. 9 illustrates a case where the inner needle 120 and the outer needle 110 protrude in an x-axis direction. FIG. 9 is a cross sectional view of the needle 110 taken along the plane passing through the central axis in the longitudinal direction of the outer needle 110.

In FIG. 9, (a) indicates the first state (charge state) where the needle tip 121 of the inner needle 120 is positioned more closely at the distal end side than the needle tip 111 of the outer needle 110 and is a state before a region in the vicinity of the capsule 2a of the prostate 2 as the biopsy region is punctured.

In FIG. 9, (b) indicates a state where only the inner needle 120 protrudes in the x-axis direction of the distal end side as the inner needle 120 is moved by the moving mechanism in order to collect tissues in the vicinity of the capsule 2a of the prostate 2. In this case, the inner needle 120 protrudes in the x-axis direction more by the above-described stroke L₂ as compared to the first state. Accordingly, the needle tip 121 of the inner needle 120 moves in the x-axis direction by the stroke L₂ from a position B₁ in the first state and reaches a position B₂ in the vicinity of the capsule 2a. Accordingly, the concave portions 123A and 123B are sufficiently exposed, and the tissue of the prostate 2 enters each inside of the concave portions 123A and 123B.

In FIG. 9, (c) indicates a state where the outer needle 110 also protrudes and the biopsy operation is ended, that is, the second state. The outer needle 110 protrudes in the x-axis direction more by the stroke L₁ as compared to the first state. The stroke L₁ is larger than the stroke L₂. Thus, the needle tip 111 of the outer needle 110 positioned at a position B₃ more closely at the proximal end side than the needle tip 121 of the inner needle 120 in the first state exceeds the concave portions 123A and 123B of the inner needle 120 and further reaches a position B₄ exceeding the needle tip 121 of the inner needle 120. Such protrusion of the outer needle 110 makes it possible to completely cover the concave portions 123A and 123B with the outer needle 110 while cutting the tissues of the prostate 2 entered the concave portions 123A and 123B using the needle tip 111 of the outer needle 110 as well as storing the tissues 2b within the concave portions 123A and 123B.

In this manner, the stroke L₁ in the longitudinal direction of the outer needle 110 is set to be larger than the stroke L₂ in the longitudinal direction of the inner needle 120 in the biopsy needle 100 according to the embodiment to enable the switching between the first state, which is the charge state where the needle tip 121 of the inner needle 120 is positioned more closely at the distal end side than the needle tip 111 of the outer needle 110, and the second state where the inner needle 120 and the outer needle 110 slide in the distal end direction and the needle tip 111 of the outer needle 110 is positioned more closely at the distal end side than the needle tip 121 of the inner needle 120, and enable the body tissue to be reliably collected in the concave portions 123A and 123B. In addition, in the biopsy needle 100, the protruding length of the inner needle 120 during the biopsy is set to be shorter than the related art as described above. Thus, the possibility that the needle tip 121 of the inner needle 120 outwardly penetrates the capsule 2a of the prostate 2 from the inner side is also remarkably lowered.

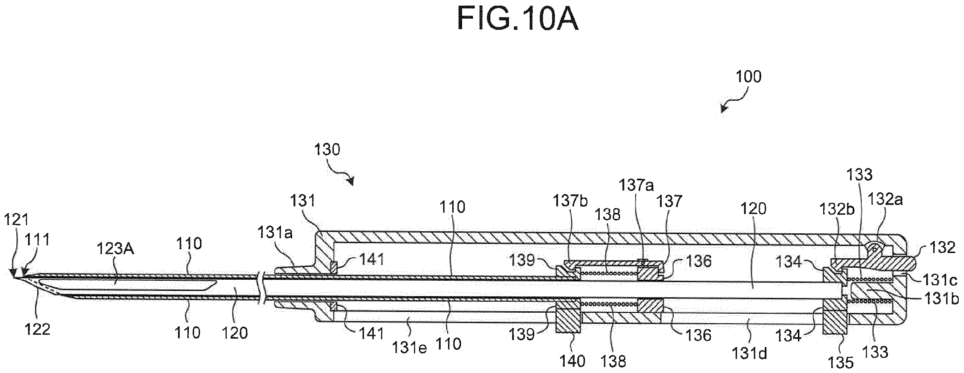

Reference will be made in detail to a biopsy operation by the biopsy needle 100. FIGS. 10A to 10F are schematic views illustrating the biopsy operation of the biopsy needle 100, and are cross sectional views obtained by cutting the distal end of the biopsy needle 100 by the plane passing through the central axis in the longitudinal direction and a center of the needle tip 121 of the inner needle 120. For illustrative purposes, FIGS. 10A to 10E show the side surface of the inner needle 120 instead of the cross section.

FIG. 10A is a schematic view illustrating the first state of the biopsy needle 100 described above. As illustrated in FIG. 10A, the inner needle fixing hook 132b is caught by the concave portion of the inner needle slider 134, the outer needle fixing hook 137b is caught by the concave portion of the outer needle slider 139, and both the inner needle charge coil spring 133 and the outer needle charge coil spring 138 are compressed and charged with the energy for extending in the first state.

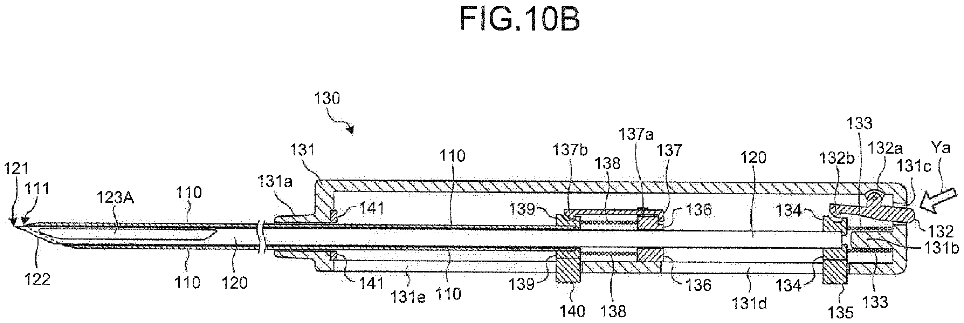

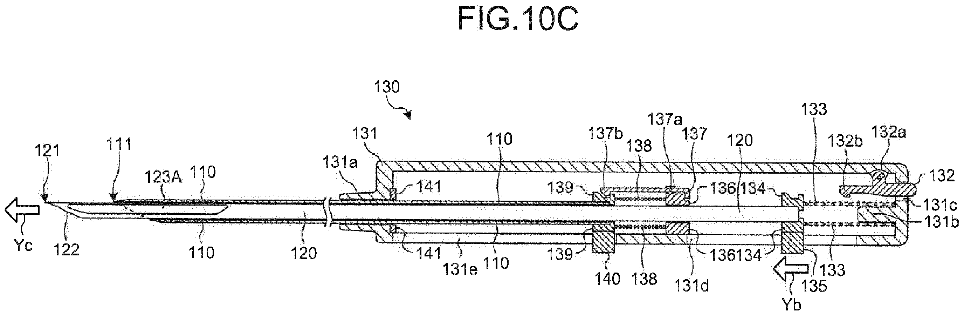

When the trigger button 132 is pressed as indicated by an arrow Ya in FIG. 10B, the inner needle fixing hook 132b at the distal end portion move up with the fulcrum 132a as the axis and out of the concave portion of the inner needle slider 134, the inner needle charge coil spring 133 that has been compressed extends in the distal end direction as illustrated in FIG. 10C, and the inner needle slider 134 is biased due to the extending of the inner needle charge coil spring 133 and slides in the distal end direction as indicated by an arrow Yb. Accordingly, the inner needle 120 also slides in the distal end direction as indicated by an arrow Yc.

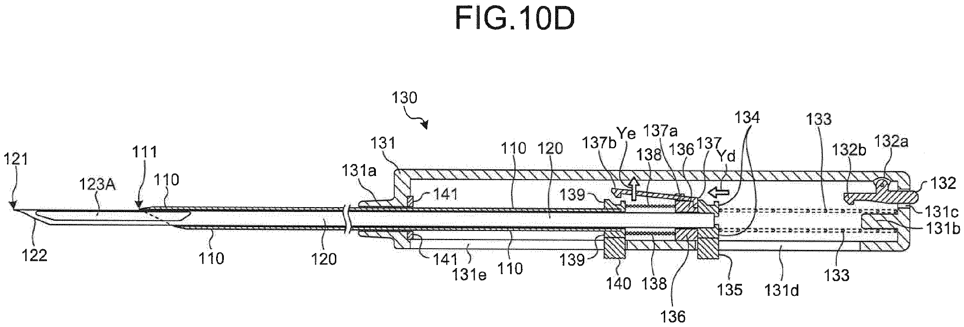

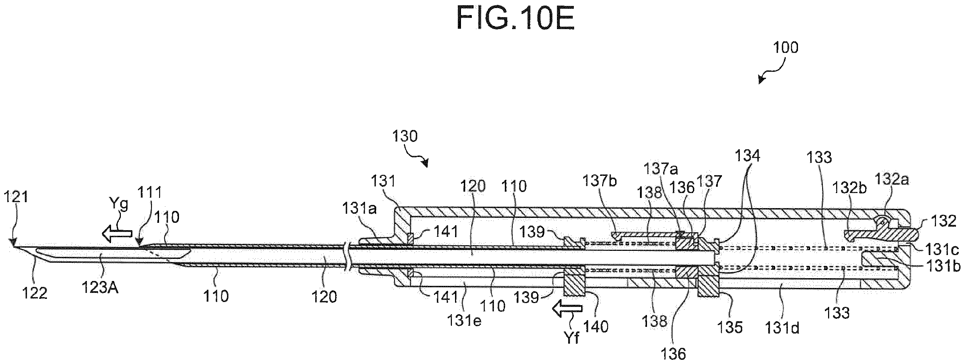

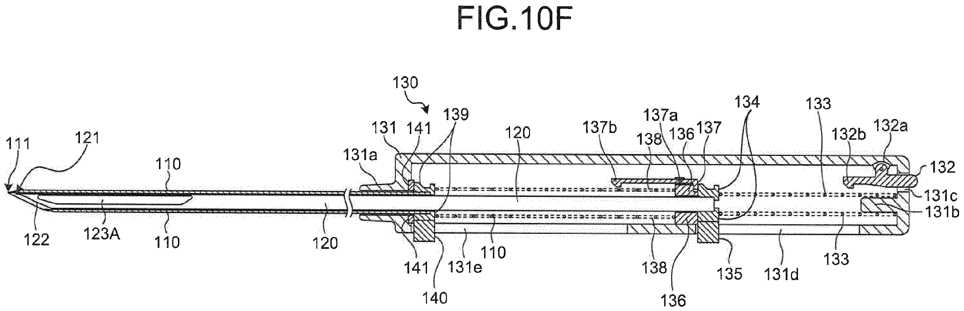

Further, the inner needle slider 134 and the inner needle knob 135 slide in the distal end direction until abutting on the inner needle stopper 136 due to the biasing of the inner needle charge coil spring 133 as illustrated in FIG. 10D. Accordingly, the inner needle 120 protrudes in the x-axis direction by the stroke L₂. Further, the inner needle slider 134 presses the outer needle fixing hook releasing lever 137 from the proximal end as indicated by an arrow Yd due to the biasing of the inner needle charge coil spring 133, the outer needle fixing hook 137b at the distal end portion moves up with the fulcrum 137a as the axis as indicated by an arrow Ye and out of the concave portion of the outer needle slider 139. Accordingly, the outer needle charge coil spring 138 that has been compressed extends in the distal end direction as illustrated in FIG. 10E, and the outer needle slider 139 and the outer needle knob 140 move in the distal end direction until abutting on the outer needle stopper 141 as indicated by an arrow Yf as being biased by the extending of the outer needle charge coil spring 138. Accordingly, the outer needle 120 protrudes in the x-axis direction by the stroke L₁ as indicated by an arrow Yg while cutting the tissues entered the concave portions 123A and 123B using the needle tip 111, and thus the biopsy needle 100 is switched to the second state illustrated in FIG. 10F.

The operator of the biopsy needle 100 pulls out the biopsy needle 100 from the biopsy region in this state. Further, the outer needle 110 is retracted (FIG. 10D) by moving the outer needle knob 140 up to the proximal end of the outer needle knob hole 131e to expose the concave portions 123A and 123B, and the body tissue inside the concave portions 123A and 123B is collected. Thereafter, the biopsy needle 100 is switched to the first state by moving the inner needle knob 135 up to the proximal end of the inner needle knob hole 131d (FIG. 10A).

In this manner, the biopsy needle 100 includes the moving mechanism in which the stroke L₁ in the longitudinal direction of the outer needle 110 is set to be longer than the stroke L₂ in the longitudinal direction of the inner needle 120 and which independently moves the outer needle 110 and the inner needle 120 to be slidable in the longitudinal direction, thereby enabling the switching between the first state and the second state.

### (Modified Example of First Embodiment)

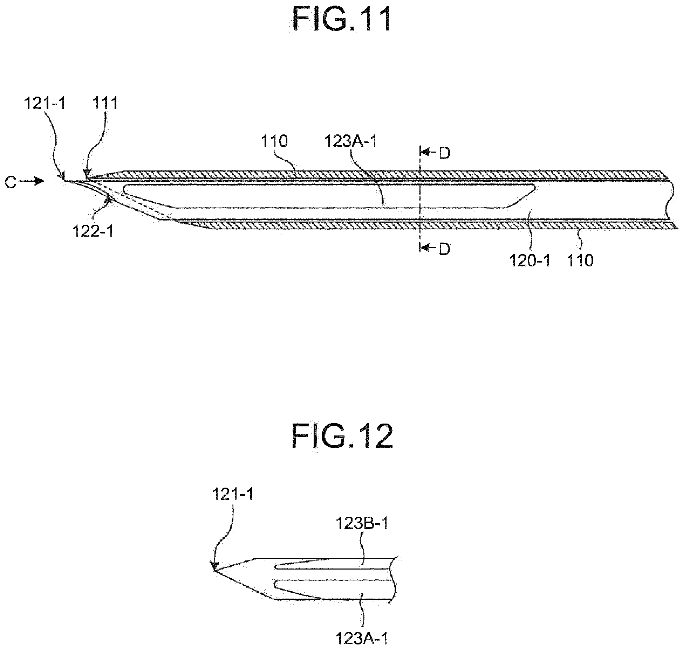

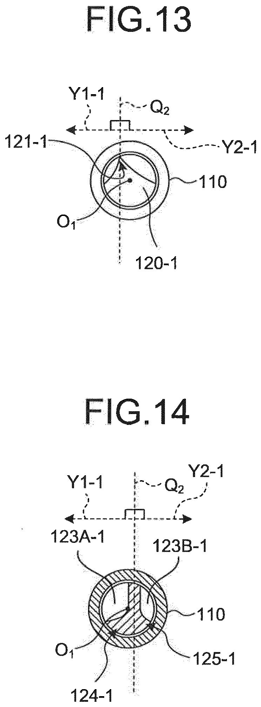

FIG. 11 is a schematic view illustrating a distal end portion of a biopsy needle according to a modified example of the first embodiment. For illustrative purposes, FIG. 11 shows a cross sectional view of an outer needle 110 taken along a plane passing through a central axis in a longitudinal direction of the outer needle 110. FIG. 12 is a perspective view of a distal end portion of an inner needle 120 illustrated in FIG. 11. FIG. 13 is a schematic view seen along an arrow C of FIG. 11. FIG. 14 is a cross sectional view taken along a line D-D of FIG. 11. As illustrated in an inner needle 120-1 in FIGS. 11 to 14, a cross section Q₂ through which a needle tip 121-1 passes does not necessarily pass through a central axis O₁ in a longitudinal direction of the inner needle 120-1, but it may be sufficient that the cross section Q₂ is a plane parallel to the longitudinal direction of the inner needle 120-1. In addition, when a direction vertical to the cross section Q₂ is defined as a first direction Y1-1 and an opposite direction to the first direction Y1-1 is defined as a second direction Y2-1, concave portions 123A-1 and 123B-1 do not necessarily have the same shape since collection of body tissues is possible as long as being provided at sides of both side surfaces of a first side surface 124-1 in the first direction Y1-1 and a second side surface 125-1 in the second direction Y2-1.

### (Second Embodiment)

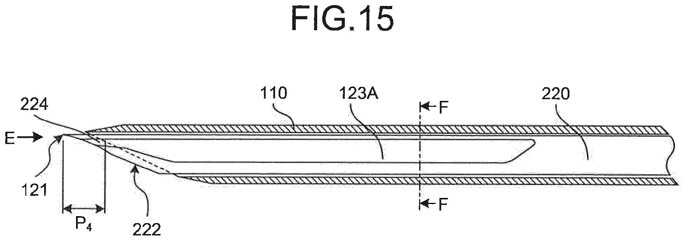

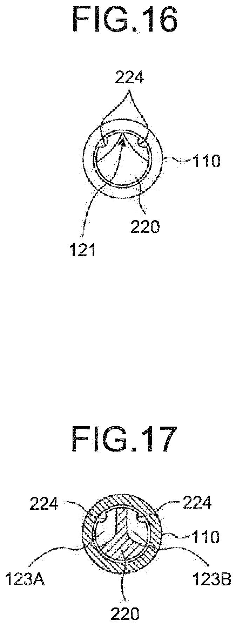

Next, a second embodiment will be described. FIG. 15 is a schematic view illustrating a distal end portion of a biopsy needle according to the second embodiment. For illustrative purposes, FIG. 15 shows a cross sectional view of an outer needle 110 taken along a plane passing through a central axis in a longitudinal direction of the outer needle 110. FIG. 16 is a schematic view seen along an arrow E of FIG. 15. FIG. 17 is a cross sectional view taken along a line F-F of FIG. 15.

As illustrated in FIGS. 15 to 17, an inner needle 220 according to the second embodiment includes two grooves 224 leading to concave portions 123A and 123B, respectively, from an inclined surface 222 at a distal end. It is possible to say that the concave portions 123A and 123B to collect body tissues are extended up to the inclined surface 222 at the distal end by the grooves 224, regarding the inner needle 220. Accordingly, it is possible to shorten a length P₄ of the inner needle 220 protruding from a collection target region in tissues, for example, up to 1 mm, and to collect body tissues very close to a needle tip 121 in the second embodiment. Thus, according to the second embodiment, it is possible to remarkably suppress a length of the needle tip 121 of the inner needle 120 coming out from the capsule 2a of the prostate 2 even in the case of collecting the body tissues in the vicinity of a capsule 2a, as compared to the first embodiment. In addition, it is also possible to house the body tissue inside each of the grooves 224 according to the second embodiment, and thus, it is possible to increase a collection amount of the body tissue as compared to the first embodiment.

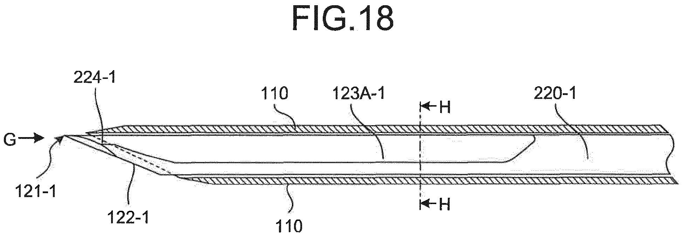

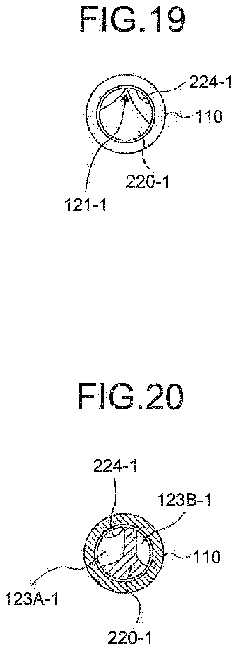

It is also possible to apply the second embodiment to a first modified example of the first embodiment. FIG. 18 is a schematic view illustrating another example of the distal end portion of the biopsy needle according to the second embodiment. For illustrative purposes, FIG. 18 shows a cross sectional view of an outer needle 110 taken along a plane passing through a central axis in a longitudinal direction of the outer needle 110. FIG. 19 is a schematic view seen along an arrow G of FIG. 18. FIG. 20 is a cross sectional view taken along a line H-H of FIG. 18. If sizes of the concave portions 123A-1 and 123B-1 are laterally different as in an inner needle 220-1 illustrated in FIGS. 18 to 20, it is possible to secure a large collection amount of body tissues only by providing a groove 224A-1 in the larger concave portion 123A-1 leading to an inclined surface 122-1.

### (Third Embodiment)

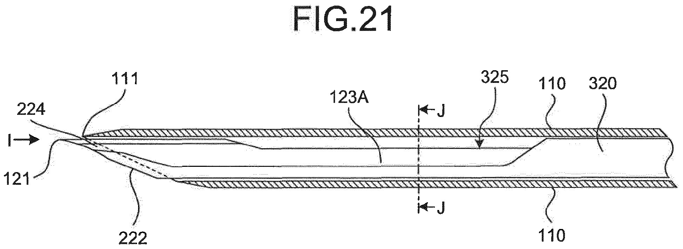

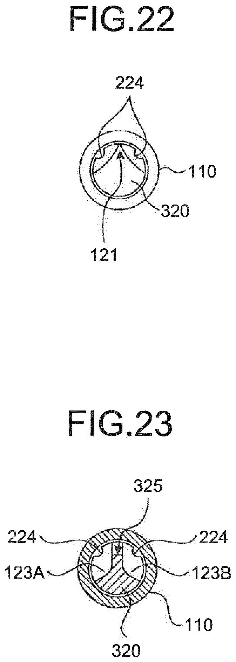

Next, a third embodiment will be described. FIG. 21 is a schematic view illustrating a distal end portion of a biopsy needle according to the third embodiment. For illustrative purposes, FIG. 21 shows a cross sectional view of an outer needle 110 taken along a plane passing through a central axis in a longitudinal direction of the outer needle 110. FIG. 22 is a schematic view seen along an arrow I of FIG. 21. FIG. 23 is a cross sectional view taken along a line J-J of FIG. 21.

As illustrated in FIGS. 21 to 23, an inner needle 320 according to the third embodiment includes two grooves 224 leading to concave portions 123A and 123B, respectively, from an inclined surface 222 at a distal end, and a cutout portion 325 obtained by cutting out a part of a portion between the two concave portions 123A and 123B in the longitudinal direction. The cutout portion 325 links the two concave portions 123A and 123B with each other. The cutout portion 325 is located on the same side as a needle tip 121 in a transverse direction of the inner needle 320.

According to the third embodiment, it is possible to increase a collection amount of body tissues by the amount obtained through the formation of the cutout portion 325 as compared to the second embodiment. Further, since the two concave portions 123A and 123B link with each other via the cutout portion 325 according to the third embodiment, it is possible to take out almost all the collected body tissues from one of the concave portions 123A and 123B, thereby achieving an effect that work of taking out the body tissue from the biopsy needle is completed at once.

### (Fourth Embodiment)

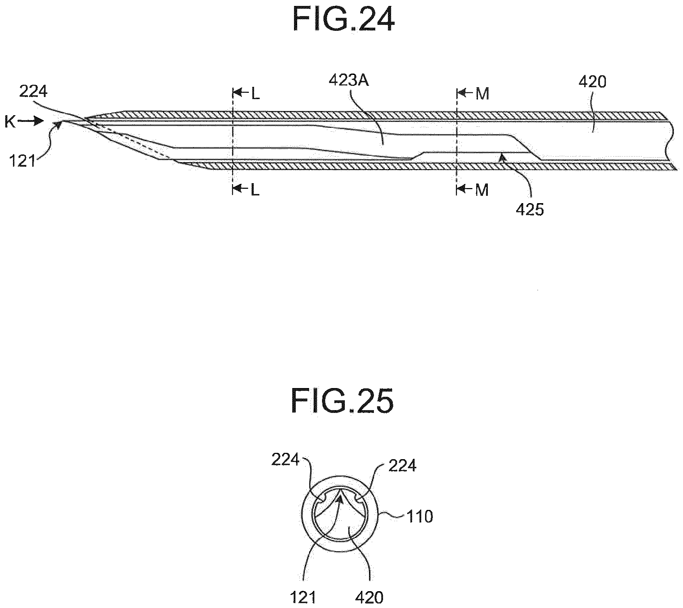

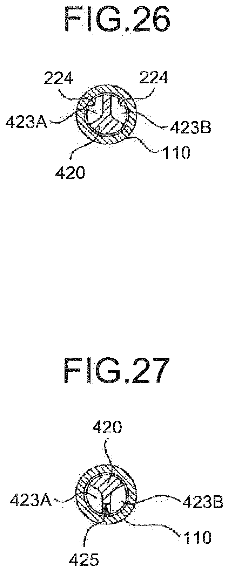

Next, a fourth embodiment will be described. FIG. 24 is a schematic view illustrating a distal end portion of a biopsy needle according to the fourth embodiment. For illustrative purposes, FIG. 24 shows a cross sectional view of an outer needle taken along a plane passing through a central axis in a longitudinal direction of the outer needle. FIG. 25 is a schematic view seen along an arrow K of FIG. 24. FIG. 26 is a cross sectional view taken along a line L-L of FIG. 24. FIG. 27 is a cross sectional view taken along a line M-M of FIG. 24.

As illustrated in FIGS. 24 to 27, concave portions 423A and 423B of inner needle 420 are moved from a needle tip 121 side to the opposite side as approaching a proximal end side from a distal end side in the longitudinal direction when seen from a transverse direction of the inner needle in the biopsy needle according to the fourth embodiment as compared to the third embodiment. That is, the concave portions 423A and 423B are opened in an upward direction of the drawing at the distal end side as illustrated in FIG. 26 and are opened in a downward direction of the drawing at the proximal end side as illustrated in FIG. 27. Further, a cutout portion 425 is formed at the proximal end side in the inner needle 420 such that the two concave portions 423A and 423B link with each other as illustrated in FIG. 27. That is, the inner needle 420 includes the cutout portion 425 that is located on the opposite side to the needle tip 121 in the transverse direction of the inner needle 420, so as to link the two concave portions 423A and 423B with each other at the proximal end side.

According to the fourth embodiment, the same effects as those of the third embodiment are achieved, and it is possible to maintain the strength at the distal end side of the inner needle 420 and easily execute the insertion into a harder body tissue by providing the configuration in which the cutout portion 425 is located on the opposite side to the needle tip 121 in the transverse direction, at the proximal end side of the concave portions 423A and 423B such that the portion between the concave portions 423A and 423B is left at the same side with the needle tip 121 in the transverse direction, as compared to the third embodiment.

Although the tissues of the prostate 2 are collected using the biopsy needle 100, another tissues may be collected instead of the prostate 2. In addition, although the distal end of the biopsy needle 100 reaches the biopsy region through the treatment tool channel of the insertion portion 10 of the ultrasound endoscope in the embodiments, the biopsy needle 100 may be inserted from outside of a body without passing through the treatment tool channel of the ultrasound endoscope, depending on biopsy regions.

### Industrial Applicability

As described above, the biopsy needle according to the present invention is useful for reliably collecting the tissues and shortening the protruding length of the biopsy needle that protrudes from the collection target region in collecting the tissues.

### Reference Signs List

2 Prostate
2a Capsule
3 Urethra
4 Bladder
10 Insertion portion
11 Aperture portion
100 Biopsy needle
110, 110P Outer needle
111, 111P, 121, 121P, 121-1 Needle tip
120, 120P, 120P', 220, 220-1, 320, 420 Inner needle
122, 122P, 122P', 122-1, 222 Inclined surface
123A, 123B, 123A-1, 123B-1, 423A, 423B Concave portion
123P, 123P' Notch portion
124, 124-1 First side surface
125, 125-1 Second side surface
130 Operating unit
131 Operating unit body
131a Lure pipe sleeve portion
131b Spring assembly convex portion
131c Trigger button hole
131d Inner needle knob hole
131e Outer needle knob hole
132 Trigger button
132a, 137a Fulcrum
132b Inner needle fixing hook
133 Inner needle charge coil spring
134 Inner needle slider
135 Inner needle knob
136 Inner needle stopper
137 Outer needle fixing hook releasing lever
137b Outer needle fixing hook
138 Outer needle charge coil spring
139 Outer needle slider
140 Outer needle knob
141 Outer needle stopper
224, 224-1 Groove
325, 425 Cutout portion

## Claims

1. A biopsy needle comprising:
an outer needle having a tubular shape and comprising a first needle tip at one end in a longitudinal direction of the outer needle; and
an inner needle having a columnar shape and configured to be inserted into the outer needle and movable along the longitudinal direction,
the inner needle comprising:
a second needle tip at a distal end of the inner needle; and
concave portions provided on first and second side surfaces of the inner needle, the first side surface facing a first direction perpendicular to a cross section parallel to the longitudinal direction, the second side surface facing a second direction opposite to the first direction.

2. The biopsy needle according to claim 1, wherein the cross section passes through the second needle tip.

3. The biopsy needle according to claim 2, wherein the cross section passes through a central axis in the longitudinal direction of the inner needle.

4. The biopsy needle according to claim 1, wherein
the inner needle further comprises an inclined surface that is inclined toward the second needle tip, and
a part of the inclined surface is included in an area obtained by projecting the concave portions in a direction parallel to a transverse direction of the inner needle.

5. The biopsy needle according to claim 1, wherein a width in a radial direction of each of the concave portions is narrower at a distal end side than at a proximal end side.

6. The biopsy needle according to claim 4, wherein the inner needle further comprises a groove leading from the inclined surface to at least one of the concave portions.

7. The biopsy needle according to claim 1, wherein
the inner needle further comprises a cutout portion in which a part of the inner needle between the two concave portions is cut out in the longitudinal direction to link the two concave portions with each other.

8. The biopsy needle according to claim 1, wherein the cutout portion is located on a same side as the second needle tip in a transverse direction of the inner needle.

9. The biopsy needle according to claim 1, wherein the cutout portion is located on an opposite side to the second needle tip in a transverse direction of the inner needle.

10. The biopsy needle according to claim 1, further comprising
a moving mechanism configured to cause the outer needle and the inner needle to independently slide in the longitudinal direction, a maximum slidable distance of the outer needle in a distal end direction being larger than a maximum slidable distance of the inner needle in the distal end direction,
wherein the moving mechanism is configured to move the outer needle and the inner needle so as to switch between a first state where the second needle tip is positioned closer to a distal end side than the first needle tip and a second state where the first needle tip is positioned closer to the distal end side than the second needle tip.

11. The biopsy needle according to claim 1, wherein the inner needle has a cylindrical shape.
